# EUROPEAN PATENT SPECIFICATION

(11) **EP 1 820 025 B1**
(45) Date of publication and mention of the grant of the patent: **29.10.2008**
(21) Application number: 05823506.0
(22) Date of filing: 24.11.2005
(51) Int. Cl.: G01N 33/68

(54) **DIAGNOSTIC METHOD FOR THE FORECASTING OF TRANSPLANTED ORGAN LOOSING**
DIAGNOSEVERFAHREN ZUR VORHERSAGE DES VERLUSTS TRANSPLANTIERTER ORGANE
PROCEDE DE DIAGNOSTIC POUR LA PREDICTION DE RELACHEMENT D'ORGANE TRANSPLANTE

(30) Priority: 25.11.2004 IT FI20040243
(43) Date of publication of application: 22.08.2007
(73) Proprietor: Universita' Degli Studi di Firenze, 50121 Firenze (IT); Regione Toscana, 50129 Firenze (IT)
(72) Inventor: ROMAGNANI, Paola, I-50139 Firenze (IT); ROTONDI, Mario, I-80059 Torre Del Greco (IT); SERIO, Mario, I-50012 Bagno A Ripoli (IT)
(74) Representative: Gervasi, Gemma
(86) International application number: PCT/EP2005/056186
(87) International publication number: WO 2006/056583

(56) References cited:
- HAUSER I A ET AL: "The ligand of CXCR3, interferon-gamma inducible CXCL9/MIG, is a highly sensitive marker of acute renal allograft rejection" NAUNYN-SCHMIEDEBERG'S ARCHIVES OF PHARMACOLOGY, vol. 369, no. Suppl. 1, March 2004 (2004-03), page R147, XP008061254 & 45TH SPRING MEETING OF THE DEUTSCHE GESELLSCHAFT FUER EXPERIMENTELLE UND KLINISCHE PHARMAKOLOGIE UND; MAINZ, GERMANY; MARCH 09-11, 2004 ISSN: 0028-1298
- HU HUAIZHONG ET AL: "Elevation of CXCR3-binding chemokines in urine indicates acute renal-allograft dysfunction." AMERICAN JOURNAL OF TRANSPLANTATION : OFFICIAL JOURNAL OF THE AMERICAN SOCIETY OF TRANSPLANTATION AND THE AMERICAN SOCIETY OF TRANSPLANT SURGEONS. MAR 2004, vol. 4, no. 3, March 2004 (2004-03), pages 432-437, XP002371136 ISSN: 1600-6135
- KANMAZ TURAN ET AL: "Surveillance of acute rejection in baboon renal transplantation by elevation of interferon-gamma inducible protein-10 and monokine induced by interferon-gamma in urine." TRANSPLANTATION. 15 OCT 2004, vol. 78, no. 7, 15 October 2004 (2004-10-15), pages 1002-1007, XP002371137 ISSN: 0041-1337
- LAZZERI E ET AL: "CITOCHINE E CHEMOCHINE NELLE NEFROPATIE E NEL TRAPIANTO RENALE CYTOKINES AND CHEMOKINES IN NEPHROPATHIES AND RENAL TRANSPLANT" GIORNALE ITALIANO DI NEFROLOGIA, WICHTIG EDITORE, MILAN, IT, vol. 19, no. 6, November 2002 (2002-11), pages 641-649, XP001193775 ISSN: 0393-5590
- AKALIN E ET AL: "Gene expression analysis in human renal allograft biopsy samples using high-density oligoarray technology." TRANSPLANTATION. 15 SEP 2001, vol. 72, no. 5, 15 September 2001 (2001-09-15), pages 948-953, XP002371138 ISSN: 0041-1337
- WHITING D ET AL: "MIG/CXCL9 neutralization attenuates chronic rejection of transplanted hearts." JOURNAL OF HEART AND LUNG TRANSPLANTATION, vol. 22, no. 1S, January 2003 (2003-01), page S154, XP008061253 & TWENTY-THIRD ANNUAL MEETING AND SCIENTIFIC SESSIONS OF THE INTERNATIONAL SOCIETY FOR HEART AND LUNG; VIENNA, AUSTRIA; APRIL 09-12, 2003 ISSN: 1053-2498
- ZHAO DAVID XIAO-MING ET AL: "Differential expression of the IFN-gamma-inducible CXCR3-binding chemokines, IFN-inducible protein 10, monokine induced by IFN, and IFN-inducible T cell alpha chemoattractant in human cardiac allografts: association with cardiac allograft vasculopathy and acute rejection." JOURNAL OF IMMUNOLOGY (BALTIMORE, MD. : 1950) 1 AUG 2002, vol. 169, no. 3, 1 August 2002 (2002-08-01), pages 1556-1560, XP002371139 ISSN: 0022-1767
- BERTONI E ET AL: "HIGH PRE-TRANSPLANT SERUM LEVELS OF CXCL10 PREDICT EARLY RENAL ALLOGRAFT FAILURE HOHE CXCL10-SERUMSPIEGEL VOR TRANSPLANTATION: HINWEIS AUF EIN FRUEHES TRANSPLANTATVERSAGEN NACH NIERENTRANSPLANTATION" TRANSPLANTATIONSMEDIZIN, PABST, LENGERICH,, DE, vol. 15, no. 2, 2003, pages 91-96, XP008031756 ISSN: 0946-9648
- ITOH Y ET AL: "Time course profile and cell-type-specific production of monokine induced by interferon-gamma in Concanavalin A-induced hepatic injury in mice: comparative study with interferon-inducible protein-10." SCANDINAVIAN JOURNAL OF GASTROENTEROLOGY. DEC 2001, vol. 36, no. 12, December 2001 (2001-12), pages 1344-1351, XP008061422 ISSN: 0036-5521

## Description

### Field of the Invention

The invention is related to serum markers for diagnostic use.

### State of art

The search of new serum markers able to predict the immune response of a subject programmed to receive an organ transplantation is an important target of clinical interest.

The sole parameter used till now in the attempt to modulate the immunosuppressive therapy on the patient is the panel of reactive antibodies (PRA).

It is well known (Am. J. Transplant. 4, 1466-1474, 2204) that the increased pre-transplantation serum levels of chemokine IP-10/CXCL10, which plays an important role in the pathogenesis of acute and chronic organ rejection, are strictly related to the risk of allograph loss.

The CXCL10, together with the other CXCR3 chemokine receptor ligands, CXCL9 (Mig) and CXCL11 (I-TAC), plays a double biological role in the allograph rejection.

These chemokines induce the migration of lymphocytes, dentritic cells, macrophages and other immune cells and modulate the angiogenesis which is an other important component of inflammation.

The described biological functions of the above mentioned chemokines are the basis of organ rejection,

It is evident the importance of availability of serum markers able to give us a prevision of the immunological reactivity of a subject who undergoes organ transplantation, and if possible with greater sensitivity, than with the previously utilized markers.

### Detailed description of the invention

We discovered that elevated serum levels of the chemokine CXCL9 provoke an extremely high risk of organ rejection. The risk is statistically higher than that of elevated serum levels of IP-10. Therefore CXCL9 represents the best serum marker for prevision of organ transplantation rejection.

We would like to point out that the serum levels of the other "sister" chemokine CXCL11 are unable to give any prevision of transplantation rejection.

The reagents and the methods used for the present invention are well known, for example Enzyme Linked-Immuno-Sorbent Assay (ELISA) using anti-CXCL9 antibodies.

### Experimental part

Serum levels before transplantation of CXCL9 and CXCL11 have been measured in 213 subjects who had a follow-up of 5 years and in 17 healthy controls. In the same subjects CXCL10 serum levels were also measured for comparison. In controls the serum levels of CXCL9 were 52.4±15.3 pg/ml while in the transplanted subjects the pre-transplantation serum levels were 255.6±14.99 pg/ml. The CXCL9 levels were higher in transplanted patients who have lost the allografh comparison to those who have kept the organ (453.27±61.5 versus 241.7±15.01). The CXCL11 resulted unusable for organ rejection prevision because the above mentioned chemokine was undetectable in the majority of patients.

The survival curves according Kaplan-Meier were performed in 300 subjects divided in 4 groups on the basis of serum levels of CXCL9 (0-25 centile = <121.4 pg/ml, n=53), (25-50 centile = between 121.4 and 194.3 pg/ml, n=64), (50- 75 centile = between 194.3 and 312.3 pg/ml, n=53), (75-100 centile = >312,3 pg/ml, n=53). These curves showed a progressive reduction of survival at 5 years distance of transplanted organ proportional to the pre-transplantation levels of CXCL9, 98.1% for group 1, 100% for group 2, 96.2% for group 3 and 79.2% for group 4 (p<0.001 in total; p<0.001 group 1 versus group 4; p<0.001 group 2 versus group 4 and p<0,001 group 3 versus group 4).

To establish the relative risks of organ decreased function in the subjects with elevated levels of CXCL9 before transplantation (>312.3 pg/ml) a multivariated analysis according Cox considering allografh loss as depending variable has been performed. Age, sex of recipient, number of mismatches HLA-A, HLA-B and HLA-DR, the primitive disease, the type of immunosuppression, the number of transplantations, the time of cold ischemia, the number of rejections, age and sex of donor have been considered as co-variables in the analysis.

The result showed that serum levels of CXCL9 above 312.3pg/ml induce a significant increase of rejection risk (risk ratio 10.390; C.I. 1.646-65.575; p=0.013) in the patients.

Another co-variated analysis was performed including CXCL10 as co-variable. Once again the pre-transplantation CXCL9 serum levels above 312.3 pg/ml determined an increased risk of organ loosing in the patients (risk ratio 10.433; CJ. 1.597-68.146; p=0.01). The other co-variables did not give a statistical significance with exception of pre-transplantation serum levels of CXCL10 which, when considered in absence of CXCL9, gave an increased risk of organ loosing but significantly lower than that obtained by CXCL9. On the basis of the above reported results the pre-transplantation serum levels of CXCL9 are able to select with highest risk of developing acute and/or chromo organ rejection and consequently with the highest risk of organ loosing. These patients should be treated with more potent immunosuppressive therapies to avoid organ loss.

The diagnostic method according to invention is useful to predict acute and/or chronic rejection and consequently organ loosing in kidney, heart, liver, bone marrow, lung, pancreas or pancreatic islets and intestine transplantation.

## Claims

1. Diagnostic method for prevision of transplanted organ loosing by measurement of pre-transplantation serum levels of CXCL9 (MIG).

2. Method according claim 1 for prevision of acute rejection of transplanted organ.

3. Method according to claim 1 for prevision of chronic rejection of transplanted organ.

4. Method according to claims 1-3 in which the transplanted organ is kidney, heart, liver, bone marrow, lung, pancreas or pancreatic islets, intestine.

5. Method according to claims 1-4 in which the serum level is measured by Enzyme Linked-Immuno-Sorbent Assay (ELISA).

## Patentansprüche

1. Verfahren für die Vorhersage eines Verlusts eines transplantierten Organs, indem die Serumkonzentrationen von CXCL9 (MIG) vor der Transplantation bestimmt werden.

2. Verfahren gemäß Anspruch 1 für die Vorhersage einer akuten Abstoßung eines transplantierten Organs.

3. Verfahren gemäß Anspruch 1 für die Vorhersage einer chronischen Abstoßung eines transplantierten Organs

4. Verfahren gemäß den Ansprüchen 1-3, wobei es sich bei dem transplantierten Organ um Niere, Herz, Leber, Knochenmark, Lunge, Pankreas oder Langerhans'-Inseln und Darm handelt.

5. Verfahren gemäß den Ansprüchen 1-4, wobei die Serumkonzentration mit Hilfe eines Enzyme Linked-Immuno-Sorbent-Assay (ELISA) bestimmt wird.

## Revendications

1. Procédé de diagnostic pour la prévision de relâchement d'organe transplanté par la mesure de niveaux de sérum de pré-transplantation de CXCL9 (MIG).

2. Procédé selon la revendication 1, pour la prévision d'un rejet aigu de l'organe transplanté.

3. Procédé selon la revendication 1, pour la prévision d'un rejet chronique de l'organe transplanté.

4. Procédé selon les revendications 1 à 3, où l'organe transplanté est un rein, coeur, foie, moëlle épinière, poumon, pancréas ou îlots pancréatiques, intestin.

5. Procédé selon les revendications 1 à 4, où le niveau de sérum est mesuré par dosage immunoadsorbant lié à une enzyme (ELISA).
